# EUROPEAN PATENT APPLICATION

(11) **EP 4 111 968 A1**
(43) Date of publication of application: **04.01.2023**
(21) Application number: 21761535.0
(22) Date of filing: 26.02.2021
(51) Int. Cl.: A61B 5/1473, A61B 5/1486, G01N 27/416

(54) **SENSOR AND METHOD FOR MANUFACTURING SAME**

(30) Priority: 27.02.2020 JP 2020031665; 27.02.2020 JP 2020031668
(71) Applicant: PHC Holdings Corporation, Tokyo 105-8433 (JP)
(72) Inventor: EZAKI, Hirofumi, Ehime 791-0395 (JP); FUJIWARA, Masaki, Ehime 791-0395 (JP)
(74) Representative: Eisenführ Speiser
(86) International application number: PCT/JP2021/007494
(87) International publication number: WO 2021/172557

(57) **Abstract**

This sensor is for measuring an analyte and has a probe to be inserted inside a living body. The probe of the sensor includes a substrate, an electrode formed on the substrate, and a reagent layer that contains an oxidoreductase and that is formed on the electrode. At at least one end of the probe in the width direction, the reagent layer and the electrode are trimmed along the insertion direction of the probe toward the inside of the living body.

## Description

### Technical Field

The present disclosure relates to a sensor and a method for manufacturing the same.

### Background Art

Representative examples of electrochemical biosensors using enzyme include an electrochemical glucose sensor used for self-monitoring of blood glucose. Also, an embedded electrochemical glucose sensor for continuously or semi-continuously measuring the concentration of glucose in a living body has been developed (see PTL 1, for example).

According to PTL 1, conductive layer 1304 and sensing layer 1306 are formed at parts further inward than an end portion of dielectric layer 1308 in a direction orthogonal to an entrance direction into a living body, for example (see FIG. 13A, for example). In other words, conductive layer 1304 and sensing layer 1306 do not reach the end portion of dielectric layer 1308. According to PTL 1, conductive layer 1304 and sensing layer 1306 formed at the parts further inward than the end portion of dielectric layer 1308 are trimmed into desired shapes. In other words, according to PTL 1, conductive layer 1304 and sensing layer 1306 are not trimmed (removed) at the end portion of dielectric layer 1308.

### Citation List

### Patent Literature

PTL 1
Japanese Unexamined Patent Application Publication (Translation of PCT Application) No. 2012-519038

### Summary of Invention

### Technical Problem

There is a need for a sensor with reduced variations in performance due to a manufacturing process.

A non-limiting example of the present disclosure provides a sensor with reduced variations in performance due to a manufacturing process and a method for manufacturing the same.

### Solution to Problem

A sensor according to an example of the present disclosure is a sensor that measures an analyte including: a probe that is to be inserted into a living body, the probe including a substrate, an electrode that is formed on or above the substrate, and a reagent layer that is formed on or above the electrode, the reagent layer and the electrode being removed along an insertion direction of the probe into the living body at at least one end portion of the probe in a width direction.

A method for manufacturing a sensor according to an example of the present disclosure is a method for manufacturing a sensor that includes a probe that is to be inserted into a living body to measure an analyte, the method including: manufacturing the probe by forming an electrode on or above a substrate; forming a reagent layer on or above the electrode; and removing the reagent layer and the electrode in an insertion direction of the probe into the living body at at least one end portion of the probe in a width direction.

### Advantageous Effects of Invention

According to an example of the present disclosure, it is possible to curb variations in performance of a sensor due to a manufacturing process.

Further advantages and effects of the example of the present disclosure will become obvious from the specification and the accompanying drawings. Although each of such advantages and/or effects will be provided by some embodiments and features described in the specification and the accompanying drawings, it is not necessary that all the advantages and/or the effects be provided to obtain one or more same features.

### Brief Description of Drawings

FIG. 1 illustrates an application example of a sensor according to the present disclosure;
FIG. 2 illustrates a sectional view of the sensor;
FIG. 3 illustrates plan views of a probe;
FIG. 4A illustrates a sectional view along arrow AA in FIG. 3;
FIG. 4B illustrates a sectional view along arrow BB in FIG. 3;
FIG. 4C illustrates a sectional view along arrow CC in FIG. 3;
FIG. 5 describes a positional relationship between a reagent layer and a film;
FIG. 6 illustrates a sectional view along arrow DD in FIG. 5;
FIG. 7 illustrates a perspective view of a reagent layer part of the probe;
FIG. 8 illustrates a plan view of a distal end part of the probe;
FIG. 9 describes the positional relationship between the reagent layer and the film;
FIG. 10 illustrates a sectional view along arrow EE in FIG. 9;
FIG. 11 illustrates examples of an opening shape of the film; and
FIG. 12 describes an example of a sensor size.

### Description of Embodiments

Hereinafter, embodiments of the present disclosure will be described in detail with reference to the accompanying drawings as needed. However, there may also be a case in which unnecessarily detailed description is omitted. For example, there may be a case in which detailed description of matters that have already been known well and repeated description of substantially the same configurations are omitted. This is for avoiding the following description from becoming unnecessarily redundant and for facilitating understanding of those skilled in the art.

Note that the accompanying drawings and the following description are provided to allow those skilled in the art to sufficiently understand the present disclosure and are not intended to thereby limit the subject matter described in the claims.

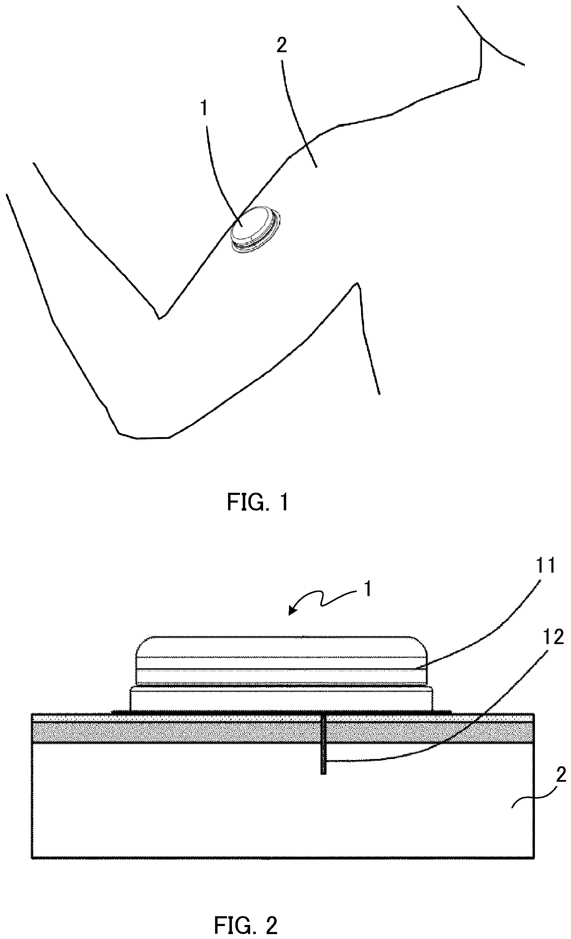

FIG. 1 illustrates an application example of sensor 1 according to the present disclosure. FIG. 1 illustrates living body 2 in addition to sensor 1. Living body 2 is, for example, a human body.

Sensor 1 illustrated in FIG. 1 is, for example, a biosensor. More specifically, sensor 1 is a CGM (Continuous Glucose Monitor) sensor. Sensor 1 is adapted such that a probe included in sensor 1 is inserted into living body 2 to continuously or semi-continuously measure glucose concentration in blood or an interstitial fluid of living body 2. For example, sensor 1 measures the glucose concentration of living body 2 for several days to several weeks.

FIG. 2 is a sectional view of sensor 1. In FIG. 2, the same reference signs are provided to the same components as those in FIG. 1.

As illustrated in FIG. 2, sensor 1 includes main body 11 and probe 12. Probe 12 is inserted into living body 2. Probe 12 includes a reagent layer containing oxidoreductase and outputs an electrical signal based on the glucose concentration to main body 11. Main body 11 stores, in a storage apparatus, the electrical signal based on the glucose concentration output from probe 12 and transmits the electrical signal to another apparatus (not illustrated) at a predetermined timing.

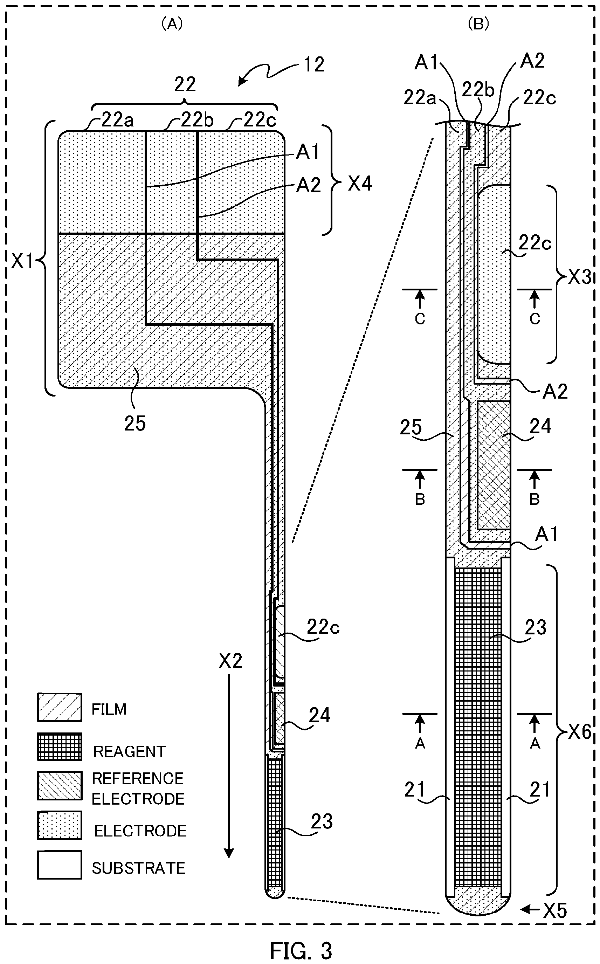

FIG. 3 illustrates plan views of probe 12. (A) of FIG. 3 illustrates entire probe 12. (B) of FIG. 3 illustrates an enlarged view of the distal end part of probe 12 illustrated in (A) of FIG. 3.

The part of region X1 (the head portion of probe 12) of probe 12 illustrated in (A) of FIG. 3 is accommodated in main body 11. The distal end part of probe 12 projects from main body 11. The distal end part of probe 12 is inserted into living body 2. Arrow X2 illustrated in (A) of FIG. 3 indicates an insertion direction of probe 12 into living body 2.

Probe 12 includes substrate 21, electrode 22, reagent layer 23, reference layer 24, and film 25.

A method for manufacturing probe 12 will be schematically described.
(1) Electrode 22 is formed on substrate 21.
   Substrate 21 is, for example, a sheet-shaped synthetic resin. Electrode 22 is uniformly formed on substrate 21.

The material of electrode 22 is, for example, gold (Au). Electrode 22 may be formed on substrate 21 by sputtering, for example. Electrode 22 may be referred to as an electrode film or an electrode layer.

(2) Electrode 22 is separated into three regions.

Grooves A1 and A2 are formed in electrode 22 formed on substrate 21 to separate electrode 22 into three regions. Electrode 22 is separated into working electrode 22a, reference electrode 22b, and counter electrode 22c by grooves A1 and A2. Grooves A1 and A2 may be formed by laser trimming, for example. Working electrode 22a may be referred to as a working electrode film or a working electrode layer. Reference electrode 22b may be referred to as a reference electrode film or a reference electrode layer. Counter electrode 22c may be referred to as a counter electrode film or a counter electrode layer.

Note that a potential (a potential with reference to the reference electrode) that is sufficient to oxidize a mediator (including not only an electronic mediator but also hydrogen peroxide) reduced by an analyte (glucose) reaction caused by oxidoreductase, for example, is provided to working electrode 22a. The glucose concentration is measured by monitoring a current flowing between working electrode 22a and counter electrode 22c.

(3) Reference layer 24 is formed.

Reference layer 24 is formed on reference electrode 22b at the distal end part of probe 12. The material of reference layer 24 is, for example, silver/silver chloride (Ag/AgCl). Reference layer 24 may be formed by a screen printing method or an ink jet method using an Ag/AgCl paste (ink), for example. Reference layer 24 may be referred to as a reference film or a reference electrode.

(4) Film 25 is disposed and fixed.

Film 25 having an opening is disposed on working electrode 22a, reference electrode 22b, counter electrode 22c, and reference layer 24 formed on substrate 21. Film 25 has a sheet shape and has an insulating property. Film 25 is disposed such that the opening part is located at the distal end part (the part forming reagent layer 23) of probe 12. A reagent, which will be described later, is dropped to the opening of film 25. Film 25 may be referred to as a film layer, an insulating layer, or an insulating film. The disposition may be referred to as lamination or placement instead.

Also, film 25 has an opening such that an upper surface (the surface in the front-side direction of the sheet surface in FIG. 3) of counter electrode 22c is partially exposed. The opening of film 25 is cut into a notch shape as illustrated in region X3 in (B) of FIG. 3 in the cutting process (7), which will be described later. With the notch shape, a part of counter electrode 22c is exposed in the upper surface. Note that the upper surface may be considered as a surface of probe 12 on a side on which reagent layer 23 is formed.

Also, film 25 has such a shape that the head portion of probe 12 is partially exposed. For example, the part of region X4 in (A) of FIG. 3 is not covered with film 25. Exposed electrode 22 in region X4 is connected to a circuit of main body 11.

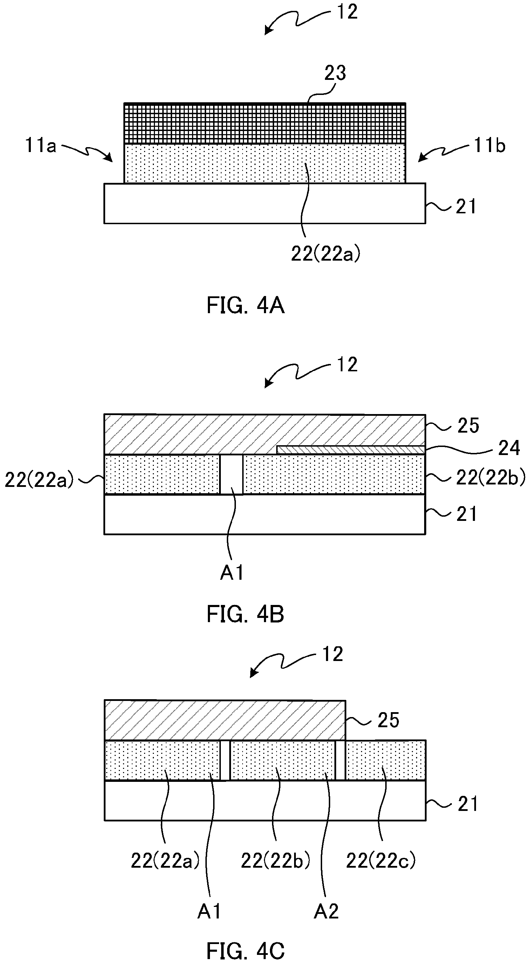

Note that the upper surface of reference layer 24 is covered with film 25 as illustrated in (B) of FIG. 3. Reference layer 24 is exposed in the width direction of probe 12 (the direction that is orthogonal to the insertion direction illustrated by arrow X2). In the example in (B) of FIG. 3, reference layer 24 is exposed to the right side surface of the distal end part of probe 12 (see reference layer 24 in FIG. 4B as well).

(5) Reagent layer 23 is formed.

Reagent layer 23 is formed on working electrode 22a at the distal end part of probe 12. For example, a reagent is dropped to the opening of film 25, which will be described above, and is then dried to thereby form reagent layer 23. It is preferable that reagent layer 23 be not formed at the distal end of probe 12 illustrated by arrow X5 in (B) of FIG. 3. In other words, reagent layer 23 is preferably formed to be separated from the distal end of probe 12. In other words, it is preferable that reagent layer 23 be not formed in a predetermined distance from the distal end of probe 12. This is because it is possible to curb peeling-off (turning-up) of reagent layer 23 from probe 12 when probe 12 is inserted into living body 2 by forming reagent layer 23 to be separated from the distal end of probe 12.

Reagent layer 23 contains at least oxidoreductase capable of causing an oxidation-reduction reaction with the analyte (glucose). Reagent layer 23 may be referred to as a reagent film, a working layer, or a working electrode.

Note that the opening of film 25 may have such a size and a shape that reagent layer 23 with a larger width than the width of probe 12, for example, is formed. Reagent layer 23 formed to have a larger width than that of probe 12 is shaped in the next trimming process.

(6) Reagent layer 23 and electrode 22 are removed.

Reagent layer 23 and electrode 22 are trimmed along the insertion direction of probe 12 at an end in the width direction of probe 12 with the outer shape formed in the cutting process (7), which will be described later. The upper surface of substrate 21 is partially exposed as illustrated in region X6 in (B) of FIG. 3 through the trimming. For the trimming of reagent layer 23 and electrode 22, laser trimming, for example, may be used.

Note that in (B) of FIG. 3, film 25 is also partially (a little) trimmed at both end parts of reagent layer 23 in the insertion direction.

(7) Probe 12 is cut out of substrate 21 through cutting.

Substrate 21 after the above processes (1) to (6) is cut into probe 12 with the shape illustrated in (A) of FIG. 3.

The cutting position includes the trimmed part. For example, a part near the center (near the center line) of the trimmed part (the bottom part of the recess) is cut.

(8) A protective film is formed.

A liquid for forming the protective film, for example, is applied to the distal end part of cut probe 12 to form the protective film. The protective film prevents or curbs leakage of substances (mainly, oxidoreductase and the electron mediator) contained in reagent layer 23 to the outside of the protective film. The protective film has a hole that transmits the analyte that is present outside the protective film into the protective film where reagent layer 23 is present. It is only necessary for the protective film to be able to protect (cover) at least the part corresponding to reagent layer 23 in probe 12.

FIG. 4A is a sectional view along arrow AA in FIG. 3. As illustrated in FIG. 4A, working electrode 22a is formed on (the upper surface of) substrate 21 at the part of probe 12 where reagent layer 23 is formed. Reagent layer 23 is formed on working electrode 22a.

Reagent layer 23 and working electrode 22a are removed at both ends of probe 12 in the width direction (side surfaces of probe 12) in the trimming process (6) described above. In the cutting process (7) described above, substrate 21 exposed in the trimming process (6) described above is cut at a position separated from reagent layer 23 and working electrode 22a. In this manner, the side surfaces of probe 12 has stepped shapes as illustrated by arrows A11a and A11b in FIG. 4A.

Note that the protective film is formed in the surroundings of the distal end part of probe 12 at reagent layer 23. In FIG. 4A, illustration of the protective film is omitted.

FIG. 4B is a sectional view along arrow BB in FIG. 3. As illustrated in FIG. 4B, working electrode 22a and reference electrode 22b are formed on substrate 21 at the part of probe 12 where reference layer 24 is formed. Working electrode 22a and reference electrode 22b are physically and electrically separated by groove A1.

Reference layer 24 is disposed on reference electrode 22b. Film 25 is formed on working electrode 22a, reference electrode 22b, and reference layer 24. Reference layer 24 includes the upper surface covered with film 25 and includes a side surface (the right side surface in FIG. 4B) of probe 12 exposed.

Note that film 25 at the upper portion of reference layer 24 may not be provided. In other words, the upper surface of reference layer 24 may be exposed.

FIG. 4C is a sectional view along arrow CC in FIG. 3. As illustrated in FIG. 4C, working electrode 22a, reference electrode 22b, and counter electrode 22c are formed on substrate 21 at the part where the upper surface of counter electrode 22c is exposed. Working electrode 22a and reference electrode 22b are physically and electrically separated by groove A1. Reference electrode 22b and counter electrode 22c are physically and electrically separated by groove A2.

Film 25 is formed on working electrode 22a and reference electrode 22b. Film 25 is not disposed on counter electrode 22c, and the upper surface of counter electrode 22c is exposed.

Examples of each component will be described.

### • Substrate 21

Substrate 21 is a synthetic resin on a sheet. For example, polyethylene terephthalate (PET) may be used for substrate 21. However, the resin material is not particularly limited as long as the resin material has at least one or more features of flexibility, easiness of working, and heat resistance like a plastic material. Other examples include general-purpose plastic such as polyethylene, polypropylene, and polyethylene naphthalate. In a case in which high heat resistance is needed, polyimide is preferably used.

### • Electrode 22

As a material of electrode 22, gold may be used as described above. However, the material is not particularly limited as long as the material is a metal or carbon material with electrical conductivity and stability (for example, it is unlikely to be oxidized or has salinity tolerance). Examples of the material of electrode 22 include platinum, palladium, and carbon.

In a case in which a metal material is used for electrode 22, the metal material may be deposited (including sputtering) on substrate 21. Other formation methods include printing, plating, and spin coating.

In a case in which carbon is used for electrode 22, electrode 22 may be formed by printing a carbon paste. In a case in which one of the upper surface and the back surface of probe 12 is caused to serve as a working electrode and the other surface is caused to serve as a counter electrode, different electrode materials may be used for the working electrode and the counter electrode.

### • Reagent layer 23

Reagent layer 23 contains oxidoreductase capable of causing an oxidation-reduction reaction with at least the analyte as described above. If oxidoreductase is dehydrogenase, an electronic mediator is further contained. Reagent layer 23 may be a system using an electronic mediator even if oxidoreductase is oxidase. In other words, although the electronic mediator is not needed by a system that electrochemically detects hydrogen peroxide generated by the oxidation-reduction reaction of glucose caused by oxidase, electrochemical detection may also be performed using the electronic mediator. In this case, reagent layer 23 includes the electronic mediator in addition to oxidase.

For the system detecting glucose, examples of oxidoreductase include glucose oxidase and glucose dehydrogenase. In regard to glucose dehydrogenase, it is desirable to use flavin adenine dinucleotide (FAD)-bound glucose dehydrogenase, and for example, enzymes derived from the genus Aspergillus (oryzae or terreus) or the genus Mucor are preferably used, in terms of low reactivity with respect to maltose.

Examples of the electronic mediator include osmium complexes, ruthenium complexes, quinone compounds, phenazine compounds, and ferrocene compounds. Also, examples of the electronic mediator include derivatives and the like thereof.

### • Reference layer 24

As a material of reference layer 24, silver/silver chloride (Ag/AgCl) may be used as described above. Reference layer 24 may be formed by screen-printing or applying an Ag/AgCl paste (ink) on or to electrode 22 and then drying it. As another formation method, reference layer 24 may be formed by performing printing, applying, plating, or the like of silver (Ag) on electrode 22 and then performing chlorination on the surface thereof.

Note that although the example in which sensor 1 according to the present disclosure has the three-electrode configuration, namely the working electrode, the counter electrode, and the reference electrode for realizing highly accurate measurement has been described, sensor 1 may have a two-electrode configuration, namely the working electrode and the counter electrode.

### • Film 25

As film 25, a product obtained by attaching an adhesive sheet (an acrylic-based, rubber-based, or hot melt-based adhesive sheet, for example) to a sheet of the same material as that of substrate 21 may be used. Also, a sheet of a material that is different from that of substrate 21 may be used. The adhesive sheet may be used alone as film 25. A thermoplastic/photoplastic resist film may be used as film 25.

Film 25 preferably has a contact angle with a liquid on the film that is greater than a contact angle with a liquid at the opening, and a greater difference therebetween is more preferable, in terms of application of the reagent, for example. For example, it is desirable that the contact angle with the liquid on the film be equal to or greater than 90° and the contact angle with the liquid at the opening be equal to or less than 50°. Even if the material does not have such a contact angle, it is also possible to cause the material to have the contact angle by performing at least one of a water repellent treatment on the film surface and a hydrophilic treatment on the opening.

Film 25 has a thickness of equal to or greater than 1 µm and equal to or less than 150 µm, preferably has a thickness of equal to or greater than 3 µm and equal to or less than 50 µm, and more preferably has a thickness of equal to or greater than 5 µm and equal to or less than 30 µm. Film 25 may be formed by printing a resist ink.

### • Protective film

Probe 12 having reagent layer 23 is used by being inserted into living body 2. Therefore, the protective film covering the surface of reagent layer 23 preferably has living body adaptability with which protein and cells are not adsorbed thereto or are unlikely to be adsorbed thereto. In general, the protective film is preferably formed of a polymer having characteristics as described above.

Examples of the polymer include a copolymer of methyl methacrylate and hydroxyethyl methacrylate, a copolymer of butyl methacrylate and hydroxyethyl methacrylate, and poly(2-methacryloyloxyethyl phosphorylcholine-co-n-butyl methacrylate). Note that it is also possible to use a (meth)acrylate-based compound having a similar principal chain to that of these exemplified polymers and having, as a side chain, a reactive group capable of causing a reaction with a linker as an "ethylene-based polymer" having a methacryloyl group or an acryloyl group, which is exemplified as a specific example of a high-molecular-weight polymer.

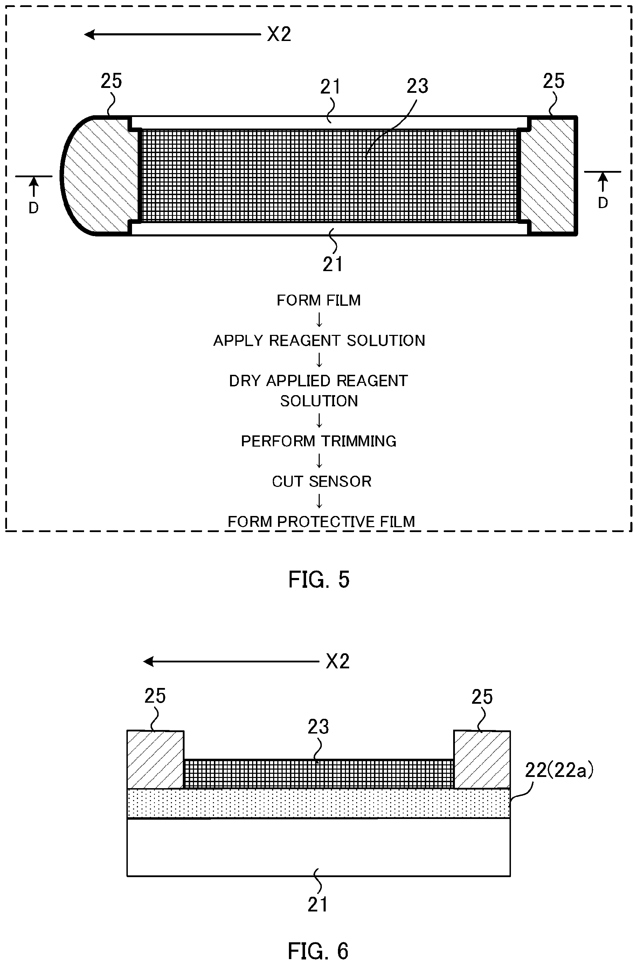

FIG. 5 describes a positional relationship between reagent layer 23 and film 25. FIG. 5 illustrates a plan view of the distal end part of probe 12. In FIG. 5, the same reference signs are provided to the same components as those in FIG. 3.

FIG. 5 illustrates a part of a process of manufacturing probe 12. "Formation of film" illustrated in FIG. 5 corresponds to the aforementioned process (4). "Apply reagent solution" and "dry applied reagent solution" correspond to the aforementioned process (5). "Perform trimming" corresponds to the aforementioned process (6). "Cut sensor" corresponds to the aforementioned process (7). "Form protective film" corresponds to the aforementioned process (8).

Note that in FIG. 5, description of the aforementioned processes (1) to (3) is omitted. The process "form film" follows the aforementioned processes (1) to (3). Also, illustration of the protective film is omitted in FIG. 5.

FIG. 6 is a sectional view along the arrow DD in FIG. 5. Film 25 having the opening is disposed on working electrode 22a. The reagent is dropped onto the opening part of film 25 and is then dried. In this manner, reagent layer 23 is formed with reagent layer 23 interposed in film 25 in the insertion direction of probe 12 as illustrated in FIG. 6. In other words, reagent layer 23 is formed with reagent layer 23 accommodated in a region defined by the opening of film 25. In other words, film 25 is adjacent to reagent layer 23 on electrode 22.

Note that film 25 on the side of the insertion direction may not be formed. For example, film 25 on the left side illustrated in FIGS. 5 and 6 may be omitted.

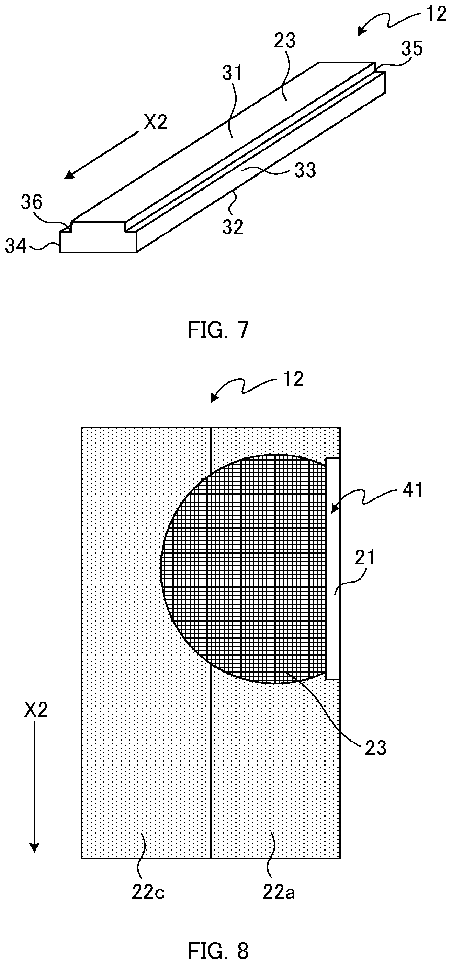

FIG. 7 illustrates a perspective view of the part corresponding to reagent layer 23 of probe 12. As illustrated in FIG. 7, probe 12 includes upper surface 31 on which reagent layer 23 is formed, back surface 32 that faces upper surface 31, side surface 33 that connects upper surface 31 to back surface 32, and side surface 34 that faces side surface 33 and connects upper surface 31 to back surface 32. Arrow X2 illustrated in FIG. 7 indicates the insertion direction of probe 12 into living body 2.

End portions of upper surface 31 of probe 12 in the width direction have stepped trimmed portions 35 and 36 from which reagent layer 23 and electrode 22 have been removed. Trimmed portions 35 and 36 are formed with a positional relationship with which they are in contact at least with reagent layer 23. In other words, reagent layer 23 extends from an end to the other end in the width direction of upper surface 31 to form upper surface 31 of probe 12 and also forms a part of the side surfaces of probe 12 (see reagent layer 23 in FIG. 4A as well).

Sensor 1 described above may be regarded as including the following components.

Sensor 1 includes main body 11 and probe 12. Probe 12 is inserted into living body 2 and acquires an electrical signal for continuously or semi-continuously measuring an analyte.

Substrate 21 includes a first surface (for example, upper surface 31) and a second surface (for example, back surface 32) facing the first surface. Also, substrate 21 includes a third surface and a fourth surface (for example, side surfaces 33 and 34) that are surfaces connecting the first surface to the second surface and extending in the insertion direction of probe 12.

Working electrode 22a is formed of a first electrode material on the first surface of substrate 21.

Reagent layer 23 is disposed at a part of working electrode 22a.

Trimmed portions 35 and 36 are formed at both end portions of the first surface in a direction that is orthogonal to the direction along the insertion direction of probe 12 into living body 2 by reagent layer 23 and the first electrode material being removed.

Reagent layer 23 contains oxidoreductase. Trimmed portions 35 and 36 are formed with a positional relationship with which they are in contact at least with reagent layer 23.

Film 25 is adjacent to reagent layer 23 in a direction opposite to the distal end side of probe 12 in the insertion direction into living body 2.

Reagent layer 23 does not have a part interposed between electrode 22 and film 25. In other words, film 25 is not disposed on reagent layer 23. Film 25 may or may not be adjacent to reagent layer 23 on the distal end side of probe 12. In other words, film 25 may or may not be formed on the distal end side of probe 12.

Sensor 1 described above may be regarded as including the following manufacturing process.

First, substrate 21 (substrate sheet) with working electrode 22a of the first electrode material formed on the first surface is prepared.

Next, a reagent solution containing oxidoreductase is applied to a predetermined position on the first surface.

Then, the reagent solution is dried to thereby form reagent layer 23.

Next, the predetermined positions of reagent layer 23 on substrate 21 are trimmed to form trimmed portions, from which reagent layer 23 and working electrode 22a formed below reagent layer 23 have been removed.

Next, substrate 21 is cut into a predetermined shape (the shape of probe 12 illustrated in (A) in FIG. 3). The position at which substrate 21 is cut includes trimmed portions 35 and 36.

Note that a protective film may be formed at the distal end part of probe 12 where reagent layer 23 is formed. The protective film includes a hole that can transmit at least an analyte (glucose) therethrough.

Also, counter electrode 22c may be formed on the first surface of substrate 21 or may be formed on the second surface. Another counter electrode (second counter electrode) that is different from the counter electrode 22c may be formed on both or one of the first surface and the second surface of substrate 21.

Also, reference electrode 22b may be formed on at least one of the first to fourth surfaces. In a case in which reference layer 24 is formed on the first surface, film 25 may be disposed on the upper surface with the third surface side exposed.

Also, reagent layer 23 may not be formed in a predetermined distance from the terminal end side (the distal end of probe 12) of the first surface in the insertion direction of probe 12.

A part (end portion) of reagent layer 23 may be interposed between electrode 22 and film 25. Reagent layer 23 may not have a part interposed between electrode 22 and film 25.

As described above, sensor 1 includes probe 12 that is to be inserted into living body 2 to measure an analyte. Probe 12 includes substrate 21, electrode 22 that is formed on substrate 21, and reagent layer 23 that contains oxidoreductase and is formed on electrode 22. Reagent layer 23 and electrode 22 of probe 12 are trimmed at least one of end portions in the width direction along the insertion direction of probe 12 into living body 2.

In this manner, variations of performance of sensor 1 caused by the manufacturing process are reduced. For example, even if a so-called coffee ring state in which reagent layer 23 dropped onto electrode 22 is thicker at its edge part than at its center portion is achieved, it is possible to use a uniform (even) part inside the ring as reagent layer 23 by the trimming.

Also, it is possible to prevent a blade tip from coming into contact with reagent layer 23 at the time of the cutting into the shape of probe 12 and to reduce cracking of reagent layer 23.

Also, it is possible to prevent the blade tip from coming into contact with reagent layer 23 at the time of the cutting into the shape of probe 12 and to reduce contamination of the reagent.

As described above, probe 12 included in sensor 1 is manufactured by a process of forming electrode 22 on substrate 21, a process of forming reagent layer 23 containing oxidoreductase on electrode 22, and a process of trimming reagent layer 23 and electrode 22 from at least one of end portions of probe 12 in the width direction along the insertion direction of probe 12 into living body 2.

In this manner, variations in performance of sensor 1 caused by the manufacturing process are reduced. For example, even if reagent layer 23 dropped onto electrode 22 is brought into the coffee ring state, it is possible to use a uniform (even) center part inside the ring as reagent layer 23 by the trimming.

It is possible to prevent the blade tip from coming into contact with reagent layer 23 by the trimming at the time of the cutting into the shape of probe 12 and thereby to reduce cracking of reagent layer 23. Also, it is possible to reduce contamination of the reagent.

As described above, film 25 is disposed on electrode 22 such that it is adjacent to reagent layer 23 at both end portions of reagent layer 23 in the insertion direction.

In this manner, variations in performance of sensor 1 caused by the manufacturing process are reduced. For example, it is possible to determine, by film 25, the position at which the reagent is to be dropped and to form uniform reagent layer 23 before the trimming.

### (Modification Example 1)

Probe 12 may have a trimmed portion at one of the ends in the width direction. In other words, there may be one trimmed portion.

FIG. 8 illustrates a plan view of the distal end part of probe 12. In FIG. 8, the same reference signs are provided to the same components as those in FIG. 3. FIG. 8 illustrates an example in which working electrode 22a and counter electrode 22c are formed in an aligned manner in the width direction of probe 12. In FIG. 8, illustration of film 25 is omitted.

Reagent layer 23 is formed to cross over the width of probe 12 on one end side of probe 12 in the width direction. Reagent layer 23 is formed not to cross over the width of probe 12 on the other end side of probe 12 in the width direction. In the example of FIG 8, reagent layer 23 is formed to cross over the right end of probe 12 and is formed not to cross over the left end of probe 12, for example.

Probe 12 includes trimmed portion 41. Trimmed portion 41 is formed on the side on which reagent layer 23 crosses over the width of the probe (the right side in FIG. 6). Trimmed portion 41 is formed by trimming reagent layer 23 and working electrode 22a. Substrate 21 is exposed by trimmed portion 41.

In this manner, probe 12 may have the trimmed portion at one of the ends in the width direction. This also leads to reduction of variations in performance of sensor 1 caused by the manufacturing process.

### (Modification Example 2)

Reagent layer 23 may stick out of the region defined by film 25 in the insertion direction of probe 12.

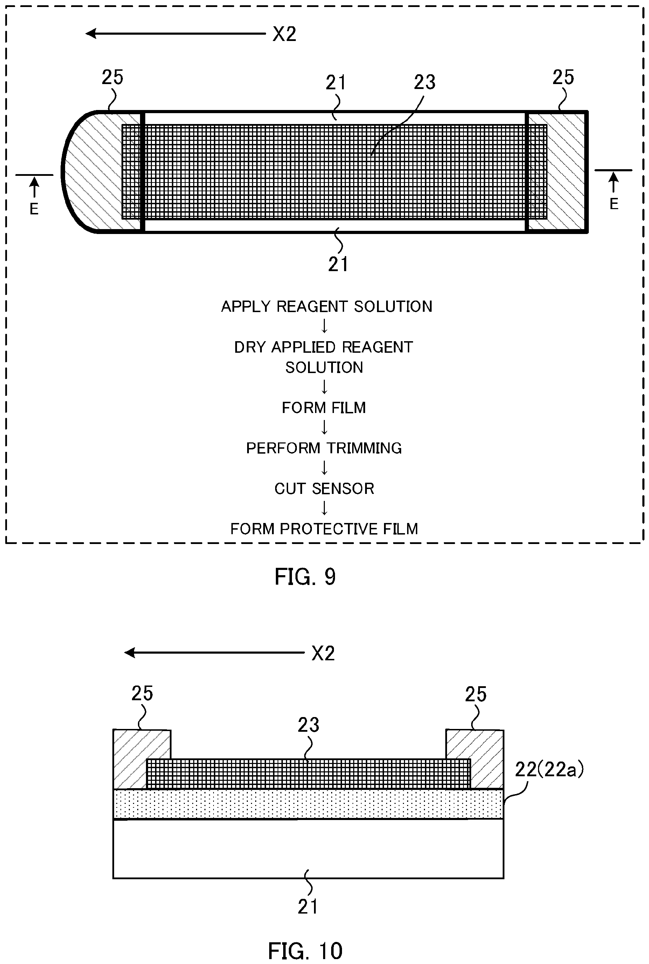

FIG. 9 is a diagram for describing a positional relationship between reagent layer 23 and film 25. FIG. 9 illustrates a plan view of the distal end part of probe 12. In FIG. 9, the same reference signs are provided to the same components as those in FIG. 3.

FIG. 9 illustrates a part of the process of manufacturing probe 12. "Apply reagent fluid" and "dry applied reagent" illustrated in FIG. 9 correspond to the aforementioned process (5). "Form film" corresponds to the aforementioned process (4). "Perform trimming" corresponds to the aforementioned process (6). "Cut sensor" corresponds to the aforementioned process (7). "Form protective film" corresponds to the aforementioned process (8).

Note that in FIG. 9, description of the aforementioned processes (1) to (3) is omitted. The process "apply reagent solution" illustrated in FIG. 9 follows the aforementioned processes (1) to (3). Also, illustration of the protective film is omitted in FIG. 9.

FIG. 10 illustrates a sectional view along arrow EE in FIG. 9. Film 25 having an opening is disposed on reagent layer 23. Film 25 is disposed such that the opening part is located at reagent layer 23. The opening of film 25 is formed to overlap reagent layer 23 at both ends of reagent layer 23 in the insertion direction (the direction of arrow X2). In other words, a part of film 25 overlaps reagent layer 23 at both ends of reagent layer 23 in the insertion direction. Also, a part of film 25 overlaps the trimmed portions at both ends of the trimmed portions in the insertion direction.

Note that film 25 on the side of the insertion direction may not be formed. For example, film 25 on the left side illustrated in FIGS. 9 and 10 may be omitted.

In this manner, film 25 is disposed on electrode 22 to overlap reagent layer 23 and trimmed portions at both end portions of reagent layer 23 in the insertion direction.

In this manner, variations in performance of sensor 1 caused by the manufacturing process are reduced. For example, it is possible to cover the end portions (the edge parts of the coffee ring) of reagent layer 23 in the insertion direction of probe 12 with film 25 and expose the uniform part of reagent layer 23.

### (Modification Example 3)

Examples of the opening shape of film 25 will be described.

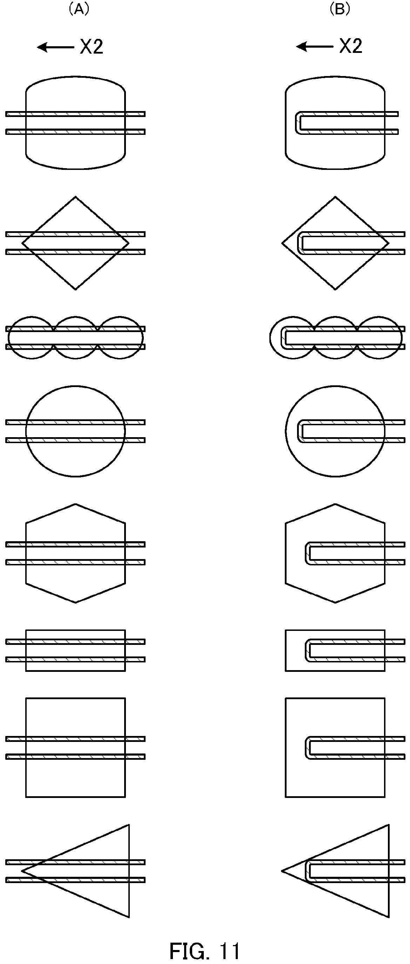

FIG. 11 illustrates examples of the opening shape of film 25. The hatched parts in (A) of FIG. 11 and (B) of FIG. 11 illustrate trimmed portions. The figures with polygonal shapes, circular shapes, and the like illustrated in (A) of FIG. 11 and (B) of FIG. 11 illustrate the shapes of the opening part of film 25. Arrow X2 illustrated in FIG. 11 indicates the insertion direction of probe 12 into living body 2.

In (A) of FIG. 11, film 25 is formed at both ends of reagent layer 23 in the insertion direction (see FIGS. 5 and 6, for example). In (B) of FIG. 11, film 25 is formed at an end on a side opposite to the distal end side of reagent layer 23 (film 25 is formed on the right side in FIGS. 5 and 6 and film 25 on the left side is not formed, for example). In this manner, the opening shape of film 25 may be various shapes.

### (Modification Example 4)

An example of the size of sensor 1 will be described.

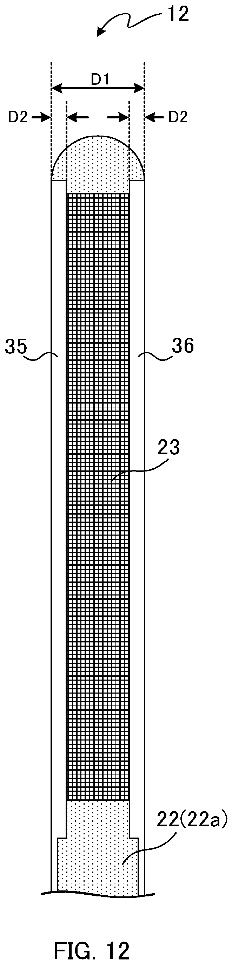

FIG. 12 describes an example of the size of sensor 1. In FIG. 12, the same reference signs are provided to the same components as those in FIGS. 3 and 7. In FIG. 12, illustration of film 25 is omitted.

Width D1 of distal end part of probe 12 is, for example, equal to or greater than 70 µm and equal to or less than 1700 µm. Width D1 is preferably equal to or greater than 70 µm and equal to or less than 600 µm and is more preferably equal to or greater than 70 µm and equal to or less than 400 µm.

Width D2 of trimmed portions 35 and 36 is, for example, equal to or greater than 5 µm. Width D2 is not particularly limited as long as a condition of a width with which it is possible to secure reagent layer 23 with respect to the width of the distal end part of probe 12 is met. In a case in which it is desired to widen width D2 of trimmed portions 35 and 36, it can be realized by performing irradiation with a laser a plurality of times.

Although the embodiments have been described with reference to the accompanying drawings, the present disclosure is not limited to such examples. It is obvious for those skilled in the art that various modification examples or amendment examples can be achieved within the scope described in the claims. It should be understood that such modification examples and amendment examples also belong to the technical scope of the present disclosure. Also, components in the embodiments may be arbitrarily combined without departing from the gist of the present disclosure.

The entire content disclosed in the specifications, the accompanying drawings, and the abstracts included in Japanese Patent Application No. 2020-031665 filed February 27, 2020 and Japanese Patent Application No. 2020-031668 filed February 27, 2020 are incorporated herein.

### Industrial Applicability

The present disclosure is suitable for use in a biosensor such as a CGM sensor, for example.

### Reference Signs List

1 Sensor
2 Living body
11 Main body
12 Probe
21 Substrate
22 Electrode
22a Working electrode
22b Reference electrode
22c Counter electrode
23 Reagent layer
24 Reference layer
25 Film
31 Upper surface
32 Back surface
33, 34 Side surface
35, 36 Trimmed portions

## Claims

1. A sensor that measures an analyte, comprising:
a probe that is to be inserted into a living body,
wherein the probe includes
a substrate,
an electrode that is formed on or above the substrate, and
a reagent layer that is formed on or above the electrode, and
the reagent layer and the electrode are removed along an insertion direction of the probe into the living body at at least one end portion of the probe in a width direction.

2. The sensor according to claim 1, further comprising:
a protective film that includes a hole through which the analyte is transmitted, the protective film covering the reagent layer.

3. The sensor according to claim 1,
wherein the electrode includes a reference electrode, a working electrode, and a counter electrode, and
the reagent layer is formed at least on the working electrode.

4. The sensor according to claim 3, wherein the counter electrode is formed on or above at least one of a first surface on the same side as a side of a surface of the substrate on or above which the reagent layer is formed, and a second surface of the substrate on a side opposite to the first surface.

5. The sensor according to claim 4, wherein the reference electrode is formed on or above at least one of the first surface, the second surface, a third surface that connects the first surface to the second surface, and a fourth surface that faces the third surface.

6. The sensor according to claim 5,
wherein the reference electrode is formed on or above the first surface,
a film is disposed on or above the reference electrode, and
the reference electrode is exposed in the third surface or the fourth surface.

7. The sensor according to claim 1, wherein the reagent layer is formed away from a distal end of the probe.

8. The sensor according to claim 1, wherein the reagent layer is formed by dropping a reagent to an opening in a film disposed on or above the electrode and drying the reagent.

9. The sensor according to claim 1, wherein a part at which the reagent layer and the electrode are removed is located at an end portion of the probe in the width direction.

10. The sensor according to claim 1, further comprising:
a film that is disposed on or above the electrode and is adjacent to the reagent layer at an end portion of the reagent layer on a side opposite to the insertion direction of the probe into the living body.

11. The sensor according to claim 10, wherein the film partially overlaps the reagent layer.

12. A method for manufacturing a sensor that includes a probe that is to be inserted into a living body and measures an analyte, the method comprising:
manufacturing the probe by
forming an electrode on or above a substrate;
forming a reagent layer on or above the electrode; and
removing the reagent layer and the electrode in an insertion direction of the probe into the living body at at least one end portion of the probe in a width direction.

13. The method for manufacturing a sensor according to claim 11, further comprising:
disposing, on or above the electrode, a film that is adjacent to the reagent layer at an end portion of the reagent layer on a side opposite to the insertion direction of the probe into the living body.
